# EUROPÄISCHE PATENTSCHRIFT

(11) **EP 3 852 853 B1**
(45) Veröffentlichungstag und Bekanntmachung des Hinweises auf die Patenterteilung: **30.08.2023**
(21) Anmeldenummer: 19813720.0
(22) Anmeldetag: 20.09.2019
(51) Int. Cl.: A61M 16/00, A61M 16/04, A61M 16/08, A61M 16/10

(54) **SYSTEM ZUR UNTERSTÜTZUNG DER ATMUNG**
SYSTEM FOR ASSISTING BREATHING
SYSTÈME D'ASSISTANCE RESPIRATOIRE

(30) Priorität: 21.09.2018 DE 102018007517
(43) Veröffentlichungstag der Anmeldung: 28.07.2021
(73) Patentinhaber: Gründler GmbH, 72250 Freudenstadt (DE)
(72) Erfinder: GRÜNDLER, Markus, 72250 Freudenstadt (DE); GRÜNDLER, Christoph, 72250 Freudenstadt (DE); RESLO, Bernd, 86934 Reichling (DE); JOOST, Thilo, 61169 Friedberg (Hessen) (DE); KÖBRICH, Rainer, 68809 Neulußheim (DE)
(86) Internationale Anmeldenummer: PCT/DE2019/000246
(87) Internationale Veröffentlichungsnummer: WO 2020/057683

(56) Entgegenhaltungen:
- EP-A1- 1 459 778
- EP-A1- 2 075 027
- WO-A1-2009/042220
- WO-A1-2013/090730
- WO-A1-2016/161092
- DE-A1-102012 223 445

## Beschreibung

Die Erfindung betrifft ein System zur Unterstützung des pulmonalen Gasaustauschs bei Patienten mit den Merkmalen des Oberbegriffs des Anspruchs 1.

Bei Atemstörungen ist die künstliche Beatmung seit Langem etabliert. Hierbei wird üblicherweise zyklisch das Atemgas mittels Überdruck über einen in die Luftröhre eingeführten Schlauch oder mittels Gesichtsmaske in die Atemwege appliziert, während die Ausatmung durch die passiven Rückstellkräfte des Atemapparates automatisch dann stattfindet, wenn der externe Überdruck abgebaut wird. Um Schädigungen durch hohe Beatmungsdrücke zu vermeiden, wird versucht, das Atemzugvolumen und die Beatmungsdrücke unter Beatmung möglichst klein zu halten. Beschränkend wirkt sich dabei stets das sogenannte "Pendelvolumen" oder "Totraumvolumen" aus, das im luftleitenden System von Patient und Beatmungssystem bidirektional bewegt wird. Das im Pendelvolumen verbleibende ausgeatmete Volumen wird beim darauffolgenden Atemzug erneut eingeatmet, der Anteil von Frischluft nimmt also mit zunehmendem Pendelvolumenanteil ab. Je größer das Totraumvolumen im Verhältnis zum Atemzugvolumen ist, desto ineffektiver ist der Gasaustausch.

Aus der Druckschrift DE 60 2004 003 409 T2 ist ein System bekannt, das als Unterstützungssystem mit einem Beatmungssystem gekoppelt ist. Das Beatmungssystem weist eine Patientenleitung auf, die vom Y-Stück in die Luftröhre eines Patienten reicht. Durch eine mit der Patientenleitung verbundene Aspirationsleitung des Unterstützungssystems wird während der Endphase der Ausatmung aus der Patientenleitung ausgeatmetes Gas abgesaugt. Gleichzeitig wird an einer anderen, näher am Ventilator gelegenen Stelle der Inspirationsleitung durch eine weitere Leitung frisches Gas nachgeführt, um die Funktion des Beatmungssystems nicht zu stören. Damit wird das durch die Patientenleitung gebildete Totraumvolumen von ausgeatmeter Luft teilweise befreit. Beim darauffolgenden Einatmen wird somit weniger ausgeatmetes Gas erneut eingeatmet, sondern eher frisches Gas. Das abgesaugte Gas wird zu Beginn der nächsten Ausatmung wieder der Patientenleitung zugeführt.

Dabei spielt die Temperierung des abgesaugten Gases vom Absaugen bis zum erneuten Zuführen eine erhebliche Rolle. Insbesondere soll sich das Gas nicht wesentlich abkühlen, um einerseits den Patienten keine Wärme zu entziehen und andererseits eine Kondensatbildung im Unterstützungssystem zu vermeiden.

Zur Führung von Atemgas sind elektrisch beheizte Schläuche bekannt. Diese haben jedoch verschiedene Nachteile: Zum einen müssen erhebliche Vorkehrungen zum Schutz des Patienten und von Dritten vor den Gefahren des elektrischen Stroms getroffen werden. Zum anderen besteht teilweise eine Überhitzungsgefahr, beispielsweise wenn Abschnitte des beheizten Schlauches z.B. durch Decken, Kissen oder den Patienten versehentlich bedeckt sind.

WO 2013/090730 zeigt einen Atemschlauch beinhaltend einen Wärmetauscher.

Der Erfindung liegt daher die Aufgabe zugrunde, ein System zur Unterstützung des pulmonalen Gasaustauschs bei Patienten zu schaffen, das eine einfache und sichere Temperierung des abgesaugten und rückgeführten Gases ermöglicht.

Diese Aufgabe wird erfindungsgemäß durch das System mit den Merkmalen des Anspruchs 1 gelöst. Das erfindungsgemäße System dient der Unterstützung des pulmonalen Gasaustauschs bei Patienten und zur Kopplung an ein Beatmungssystem oder zur Anwendung bei nicht-beatmeten Patienten. Das System weist einen flexiblen Schlauch auf, der insbesondere in die Luftröhre eines Patienten einführbar ist, insbesondere bis in die distale Trachea bzw. die Hauptbronchien. Dabei kann der Schlauch durch einen operativ geschaffenen Zugang, insbesondere im Halsbereich, oder durch Mund oder Nase in die Luftröhre eingeführt sein. Insbesondere im Fall des durch ein Beatmungssystem beatmeten Patienten kann der flexible Schlauch durch einen Endotrachealtubus oder eine Trachealkanüle des Beatmungssystems in die Luftröhre eingeführt oder in den Endotrachealtubus beziehungsweise die Trachealkanüle integriert sein. Weiterhin weist das System eine bidirektional wirkende Pumpeinheit und insbesondere eine Reservoireinheit auf. Die Pumpeinheit dient dazu, Gas aus der Lunge, aus der Luftröhre oder aus dem Leitungssystem eines Beatmungsgerätes abzusaugen und wieder durch Pumpen zurück zu führen, was nachfolgend auch als "Rückführung" bezeichnet wird. Die Reservoireinheit dient der Zwischenspeicherung des abgesaugten Gases, bevor es wieder zurückgeführt wird. Die Zwischenspeicherung kann außerdem zur Temperierung des Gases genutzt werden. Die Pumpeinheit und die Reservoireinheit bilden vorzugsweise eine gemeinsame Einheit und sind insbesondere als Kolbenpumpe ausgeführt. Weiterhin weist das System insbesondere eine Steuerung derart auf, dass über den flexiblen Schlauch mittels der Pumpeinheit eine Absaugung und eine Rückführung des abgesaugten Gases einstellbar ist. Die Absaugung erfolgt insbesondere exspiratorisch und insbesondere end-exspiratorisch, während die Rückführung insbesondere spät inspiratorisch erfolgt. Dies ermöglicht den oben beschriebenen Effekt der Minderung des Totraumvolumens, da bei der Einatmung der Rückatemanteil verbrauchter Atemgase reduziert wird. In der Folge wird eine Vergrößerung der Partialdruckdifferenzen zwischen Blut- und Gasseite in der Lunge erreicht, was zu einer Verbesserung der Oxygenierung sowie zu einer erhöhten Eliminierung lungengängiger Stoffe, z.B. CO₂ oder Alkohol, führt. "Rückführung" meint eine zumindest teilweise Zurückleitung in den Patienten und zwar insbesondere durch den flexiblen Schlauch hindurch, durch den zuvor die Absaugung erfolgt ist.

Das System ist dadurch gekennzeichnet, dass der flexible Schlauch zumindest abschnittsweise von einem Hüllschlauch umfasst ist und dass innerhalb des Hüllschlauchs außerdem ein Heizschlauch zum Führen eines Mediums angeordnet ist, und zwar derart, dass das Medium durch den Heizschlauch in eine erste Richtung und zumindest teilweise außerhalb des Heizschlauchs sowie innerhalb des Hüllschlauchs in eine entgegengesetzte zweite Richtung führbar ist. Das Medium ist bevorzugt gasförmig und ist insbesondere Luft. Das Medium wird dabei insbesondere eine Temperatur oberhalb der Umgebungstemperatur aufweisen und dem Heizen des flexiblen Schlauchs dienen. Nicht ausgeschlossen ist aber grundsätzlich auch eine Kühlwirkung.

Der Hüllschlauch, der Heizschlauch und der flexible Schlauch sind insbesondere separate Schläuche, womit gemeint ist, dass sie jeweils eigene Schlauchwände aufweisen. Die Schläuche können jedoch auch gemeinsame Schlauchwände aufweisen, also als mehrlumiger Schlauch ausgeführt sein. Insbesondere können der Hüllschlauch, der Heizschlauch und der flexible Schlauch als ein einziger, mehrlumiger Schlauch ausgeführt sein. Es können innerhalb eines Hüllschlauchs mehrere flexible Schläuche und/oder mehrere Heizschläuche angeordnet sein, wobei diese wiederum als ein oder mehrere mehrlumige Schläuche ausgeführt sein können. Vorzugsweise ist innerhalb des Hüllschlauchs zumindest abschnittsweise mindestens ein weiterer flexibler Schlauch angeordnet. Im Hüllschlauch können außerdem weitere sonstige Schläuche oder Leitungen, wie beispielsweise elektrische Leitungen, geführt sein.

Vorzugsweise fließt das Medium zunächst durch den Heizschlauch in die erste Richtung und dann außerhalb des Heizschlauchs zurück in die entgegengesetzte zweite Richtung. Die Reihenfolge des Durchfließens kann jedoch auch umgekehrt sein.

Das Medium dient dem Temperieren des flexiblen Schlauchs und damit dem Temperaturerhalt des in dem flexiblen Schlauch geführten Atemgases. Damit das Temperieren im Wesentlichen über die gesamte Länge des Hüllschlauchs erfolgt, erstreckt sich insbesondere der Heizschlauch mindestens über die halbe Länge des Hüllschlauchs.

Vorzugsweise weist der Heizschlauch innerhalb des Hüllschlauchs ein offenes Ende und/oder Öffnungen auf, durch die das Medium entweichen oder - bei umgekehrter Fließrichtung - einströmen kann. Ein offenes Ende ist die einfachste Geometrie, mehrere Öffnungen, insbesondere über den Umfang und die Länge verteilt, können die Verteilung des Mediums innerhalb des Hüllschlauchs verbessern. Hierdurch kann die Temperaturverteilung innerhalb des Hüllschlauchs gesteuert und damit letztlich die Temperierung des Atemgases im flexiblen Schlauch optimiert werden. Hat der Hüllschlauch eine Abzweigung, worauf im Folgenden noch eingegangen wird, so kann eine Öffnung im Bereich der Abzweigung bewirken, dass das Medium auch in die Abzweigung gelangt, insbesondere wenn die Abzweigung außerdem eine Leckageöffnung aufweist, worauf ebenfalls noch eingegangen wird.

Alternativ zu einem offenen Ende oder Öffnungen kann ein zusätzliches Element, beispielsweise eine Umlenkeinrichtung am Ende des Heizschlauchs angeordnet sein. Dieses zusätzliche Element kann auch einstückig mit einem Endstück oder Anschlussstück für den Hüllschlauch sein, wobei das Endstück insbesondere eine Durchführöffnung für den flexiblen Schlauch aufweist.

In einer bevorzugten Ausführungsform der Erfindung weist der Hüllschlauch eine Halteeinrichtung zum Halten des Heizschlauchs in einer festen Position längs des Hüllschlauchs auf. Hierdurch wird vermieden, dass sich der Heizschlauch verschiebt. Eine Verschiebung könnte bewirken, dass das Medium einen anderen Weg als vorgesehen entlang des flexiblen Schlauchs nimmt. Die Halteeinrichtung kann einstückig mit anderen Elementen sein, insbesondere mit einem Endstück oder Anschlussstück für den Hüllschlauch und/oder mit einer Umlenkeinrichtung zum Umlenken des Mediums. Es können mehrere Halteeinrichtungen längs des Hüllschlauchs vorgesehen werden.

Alternativ oder zusätzlich können Stützelemente vorgesehen sein, die die Schläuche innerhalb des Hüllschlauchs in einer festen Position bezüglich des Querschnitts halten.

Vorzugsweise weist der Hüllschlauch oder ein mit dem Hüllschlauch verbundenes Anschlussstück eine Leckageöffnung auf. Die Leckageöffnung dient dazu, dass nach dem Austritt des Mediums aus dem Heizschlauch ein Teil des Mediums aus dem Hüllschlauch oder dem Anschlussstück austreten kann, während ein anderer Teil des Mediums längs des Heizschlauchs zurückströmt. Dabei muss die Leckageöffnung nicht in unmittelbarer Nähe des offenen Endes beziehungsweise der Öffnungen des Heizschlauchs angeordnet sein, sondern das Medium kann noch ein Stück in die erste oder zweite Richtung oder auch in eine Abzweigung des Hüllschlauchs fließen. Insbesondere ist jedoch der kürzeste Abstand der Leckageöffnung zum offenen Ende oder zu Öffnungen kleiner als der Abstand zu dem Ende des Heizschlauchs, in den das Medium eingeleitet wird. Die Leckageöffnung hat den Vorteil, dass das Medium auch in Bereiche geführt werden kann, in die kein Heizschlauch führt, beispielsweise weil der Hüllschlauch sich verengt und kein Platz für einen Heizschlauch besteht, oder weil eine Abzweigung des Hüllschlauchs, in den kein Heizschlauch führt, mit dem Medium temperiert werden soll. Die Leckageöffnung stellt eine Öffnung zur Umgebung dar, weshalb gegebenenfalls Vorsichtsmaßnahmen zum Schutz vor dem austretenden Medium getroffen werden müssen.

In einer bevorzugten Ausführungsform ist der Hüllschlauch armiert, zweischichtig und/oder ein Wellschlauch, wodurch er besonders stabil ist, insbesondere gegen ungewolltes Abknicken oder Abquetschen, was sowohl den Fluss und damit die Wirkung des Mediums stören würde, als auch zu einer Störung des Flusses durch den flexiblen Schlauch führen könnte.

Vorzugsweise ist der Hüllschlauch transparent, um beispielsweise eine Fehlfunktion, wie eine Kondensatbildung, einfach erkennen zu können. Ist auch der flexible Schlauch transparent, kann außerdem auch eine Fehlfunktion oder eine Sekretanhäufung oder dergleichen innerhalb des flexiblen Schlauchs erkannt werden.

Für den Fall, dass innerhalb des Hüllschlauchs ein weiterer flexibler Schlauch angeordnet ist, die beiden flexiblen Schläuche jedoch zu unterschiedlichen Orten führen sollen, schlägt die Erfindung vor, dass der Hüllschlauch eine Abzweigung aufweist und die beiden flexiblen Schläuche insbesondere nur bis zu der Abzweigung nebeneinander geführt werden. Die Abzweigung kann als ein beispielsweise T- oder Y-förmiges Anschlussstück mit einer Durchführöffnung für den abzweigenden flexiblen Schlauch und mit oder ohne einen abzweigenden Hüllschlauch ausgeführt sein. Auch ist es möglich, dass es einen abzweigenden Heizschlauch gibt, jedoch kann die Beheizung wie oben beschrieben auch durch eine Öffnung im Heizschlauch im Bereich der Abzweigung sowie eine Leckageöffnung am Ende der Abzweigung erreicht werden.

Um die Heizwirkung durch das Medium steuern zu können, schlägt die Erfindung vor, dass ein Temperatursensor derart angeordnet ist, dass die Temperatur des Mediums im Bereich des Anfangs und/oder Endes des Wegs des Mediums messbar ist. Weitere Temperatursensoren zwischen dem Anfang und dem Ende des Wegs des Mediums können die Steuerung weiter verbessern. Wie erwähnt fließt das Medium vorzugsweise zunächst durch den Heizschlauch in die erste Richtung und dann außerhalb des Heizschlauchs zurück in die entgegengesetzte zweite Richtung. In diesem Fall ist insbesondere ein Temperatursensor im Bereich desjenigen Endes des Heizschlauchs angeordnet, wo das Medium in den Heizschlauch eintritt und ein weiterer Temperatursensor dort, wo das Medium in umgekehrter Richtung aus dem Hüllschlauch wieder austritt. Alternativ oder zusätzlich kann ein Temperatursensor im Bereich einer Leckageöffnung und/oder im flexiblen Schlauch vorgesehen werden. Über die Temperaturen an all diesen Stellen kann auf die Temperierwirkung zurückgeschlossen werden.

Der Hüllschlauch kann über seine gesamte Länge einen konstanten Außendurchmesser haben, vorzugsweise weist er jedoch Abschnitte mit unterschiedlichen Außendurchmessern auf. Insbesondere weist der Hüllschlauch eine Stufung auf, beispielsweise um in der Nähe des Patienten kleinere und flexiblere Abmessungen zu haben. Das gleiche gilt für den Heizschlauch und den flexiblen Schlauch. Auch müssen die genannten Schläuche nicht über ihre gesamte Länge aus dem gleichen Material gefertigt sein.

Der Hüllschlauch kann auch Abschnitte mit derart angepassten Querschnitt aufweisen, dass neben dem flexiblen Schlauch weitere Elemente, wie Filter, Sensoren und/oder eine Tubusverlängerung, umhüllt werden, um diese zusätzlich zu temperieren.

Das Medium kann ausgehend von einer Wärmequelle, beispielsweise einer elektrischen Heizwendel mit Lüfter, direkt in den Heizschlauch geleitet werden. Erfindungsgemäß beheizt das Medium zunächst die Pumpeinheit und dann erst wird in den Heizschlauch geleitet.

Die Wärmequelle braucht kein separates Element sein, sondern kann ein Aggregat des Systems, wie Motor der Pumpeinheit, sein, dessen Abwärme genutzt wird.

Die Erfindung wird nachfolgend anhand eines Ausführungsbeispiels erläutert. Es zeigen:
- Figur 1: eine schematische Darstellung des erfindungsgemäßen Systems;
- Figur 2: eine perspektivische Schnittdarstellung eines Bereichs vom Tubus bis zum Y-Stück;
- Figur 3: eine vergrößerte Ansicht eines ersten Ausschnitts aus Figur 2; und
- Figur 4: eine vergrößerte Ansicht eines zweiten Ausschnitts aus Figur 2.

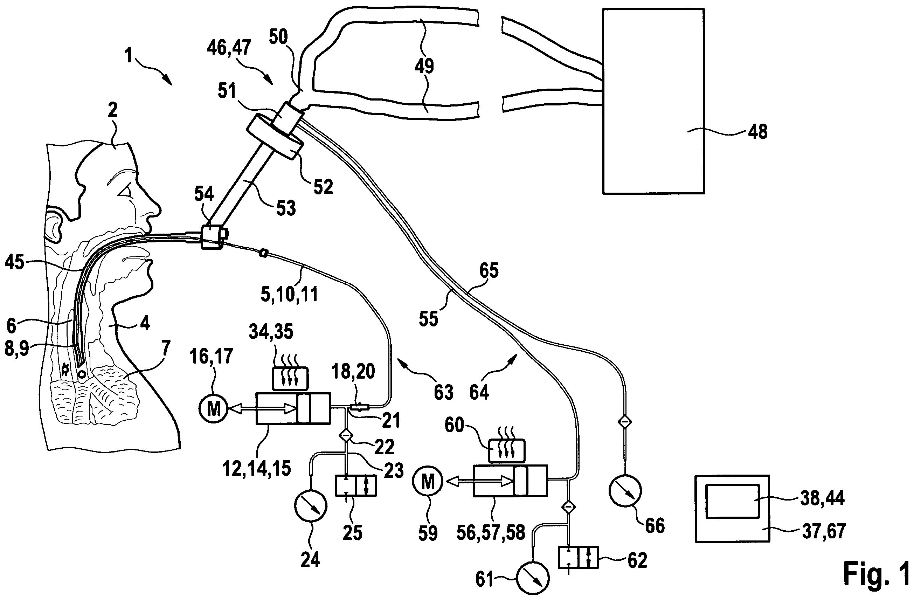

Figur 1 zeigt einen schematischen Überblick über das erfindungsgemäße System 1 in der Anwendung bei einem beatmeten Patienten 2. Durch einen schlauchartigen Tubus 45, der Teil eines Leitungssystems 46 eines Beatmungssystems 47 ist, ist ein flexibler Schlauch 5 des Systems 1 von außen in die Luftröhre 6 des Patienten 2 eingeführt worden und reicht bis nahe der Lunge 7 des Patienten 2. Der flexible Schlauch 5 besteht aus einem einen ersten Schlauchabschnitt 8 in Form eines Katheters 9 zum Einführen in die Luftröhre 6 und einen zweiten Schlauchabschnitt 10 in Form eines Verbindungsschlauchs 11 zur Verbindung des ersten Schlauchabschnitts mit einer Reservoireinheit 12 des Systems 1. Der Verbindungsschlauch 11 weist einen größeren Außenquerschnitt als der Katheter 9 auf.

Die Reservoireinheit 12 ist als eine Kolbenpumpe 14 ausgebildet und ist somit gleichzeitig eine bidirektional wirkende Pumpeinheit 15. Die Kolbenpumpe 14 wird von einem Linearmotor 16 als Antriebseinheit 17 angetrieben. Die Antriebseinheit 17 ist in Figur 1 nur symbolisch dargestellt.

Der Verbindungsschlauch 11 ist unterbrochen von einer Messküvette 18 für einen CO₂-Sensor 20, der an der Messküvette 18 angebracht ist. Zwischen der Messküvette 18 und der Kolbenpumpe 14 ist der Verbindungsschlauch 11 außerdem durch einen ersten Abzweig 21 unterbrochen. Dieser erste Abzweig 21 ist wie auch die nachfolgenden Abzweige jeweils durch ein T-Stück oder Y-Stück gebildet. Der erste Abzweig 21 führt über einen ersten Filter 22, der wie auch die nachfolgend beschriebenen Filter eine hygienische Barriere bildet, zu einem zweiten Abzweig 23, an dessen einem Ausgang ein Hochdrucksensor 24 und dessen anderem Ausgang ein Be- und Entlüftungsventil 25 mit Ausgang zur Umgebung angeschlossen ist. Der Hochdrucksensor 24 ist zum Messen von Drücken im Bereich von -750 mbar bis +750 mbar ausgelegt und dient damit der Kontrolle der Absaug- und Pumpdrücke der Kolbenpumpe 14. Die Kolbenpumpe 14 ist durch ein thermoelektrisches Heizelement 34 als Temperiereinheit 35 beheizbar.

Die Sensoren, also der CO₂-Sensor 20 und der Hochdrucksensor 24, sowie die Aktoren, also der Linearmotor 16, das Heizelement 34 sowie das Be- und Entlüftungsventil 25 sind jeweils über elektrische Leitungen, die der Übersicht halber nicht dargestellt sind, mit einer Steuerung 37 verbunden. Die Steuerung 37 umfasst insbesondere eine Ein- und Ausgabeeinheit in Form einer berührungsempfindlichen Anzeige 38, ein sogenannter "Touch-Screen", zur Bedienung durch einen Anwender.

Das Beatmungssystem 47 weist eine Zentraleinheit 48 mit Pumpe, Steuerung, Benutzerschnittstelle und dergleichen auf, worauf es hier nicht im Detail ankommt. Von dieser Zentraleinheit 48 gehen zwei Beatmungsschläuche 49 ab bis zu einem Y-Stück 50, an den sich nacheinander ein erster Konnektor 51, ein Filterelement 52 mit HME (Heat and Moisture Exchanger), eine rohrartige Tubusverlängerung 53 und schließlich ein zweiter Konnektor 54 anschließt. An den zweiten Konnektor 54 ist der Tubus 45 angeschlossen. Das Beatmungssystem 47 arbeitet in bekannter Weise, das heißt über einen Beatmungsschlauch 49, den Tubus 45 und die dazwischen liegenden Bauteile wird Luft, Sauerstoff und/oder therapeutische Gase in die Lunge 7 des Patienten 2 gepumpt und im Wechsel dazu die verbrauchte Luft in umgekehrter Richtung, aber über den anderen Beatmungsschlauch 49 wieder abgeführt. Die Beatmungsschläuche 49 bilden zusammen mit dem Tubus 45 und den dazwischen liegenden Bauteilen das Leitungssystem 46 des Beatmungssystems 47. Natürlich sind hier auch Beatmungsgeräte denkbar, die nur einen Beatmungsschlauch aufweisen und beispielsweise ein patientennahes Ventil- oder Leckagesystem.

An den ersten Konnektor 51 ist ein weiterer flexibler Schlauch 55 angeschlossen. Dieser weitere flexible Schlauch 55 dient der Verbindung mit einer weiteren Kolbenpumpe 56, die eine weitere Pumpeinheit 57 und einer weitere Reservoireinheit 58 bildet. Die weitere Kolbenpumpe 56 wird über einen weiteren Linearmotor 59 angetrieben. Der Kolbenpumpe 56 ist eine weitere Temperiereinheit 60 zugeordnet und über eine Verzweigung und einen Filter ist ein weiterer Hochdrucksensor 61 sowie ein weiteres Be- und Entlüftungsventil 62 angeschlossen. Während der zuvor beschriebene flexible Schlauch 5 mit den daran angeschlossenen Elementen, wie der Kolbenpumpe 14, einen ersten Strang 63 bildet, bildet der weitere flexible Schlauch 55 mit den hieran angeschlossenen Elementen, wie der weiteren Kolbenpumpe 56, einen zweiten Strang 64. Der zweite Strang 64 weist keine Messküvette auf, was allerdings nicht ausgeschlossen ist, wenn in diesem Bereich beispielsweise die CO₂-Konzentration gemessen werden soll.

An den ersten Konnektor 51 ist außerdem ein dritter flexibler Schlauch 65 angeschlossen, der über einen Filter mit einem Beatmungsdrucksensor 66 verbunden ist. Der Beatmungsdrucksensor 66 hat einen Messbereich bis +-100 mbar. Über die Druckdifferenz zwischen dem Hochdrucksensor 61 und Hochdrucksensor 24 kann in Momenten, in denen Gasfluss-Stillstand in den flexiblen Schläuchen 5, 55 herrscht, auf den Beatmungsfluss in Richtung zum beziehungsweise vom Patienten geschlossen werden. Dies kann auch durch pneumatische Zusammenschaltung an einem gemeinsamen Drucksensor erfolgen, der gegebenenfalls mittels Schutzventilen vor schädlichen Überdrücken während der Bewegung der Kolbenpumpen 14, 56 geschützt ist (nicht dargestellt). Dies erlaubt es, das System 1 mit dem Beatmungssystem 47 zu synchronisieren.

Auch die Komponenten des zweiten Strangs 64 sowie der Beatmungsdrucksensor 66 sind an die Steuerung 37 angeschlossen.

Die beiden Stränge 63, 64 unterstützen die Beatmung des Patienten 2, die im Wesentlichen durch das Beatmungssystem 47 erfolgt, indem mittels der beiden Kolbenpumpen 14, 56 jeweils im Wechsel abgepumpt und zurückgeführt wird. Dabei wird am Ende der Ausatmung verbrauchtes Atemgas vom ersten Strang 63 aus den Atemwegen gesaugt, während im Wesentlichen gleichzeitig vom zweiten Strang 64 frisches Gas zurückgeführt wird. Tatsächlich beginnt das Zurückführen schon etwas früher, damit im Leitungssystem 46 des Beatmungssystems 47 keine Druckminderung erfolgt, die dem Beatmungssystem ein Einatmen der Lunge suggerieren würde. Für das leicht versetzte Arbeiten der Kolbenpumpen 14, 56 ist es entscheidend, dass diese nicht starr gekoppelt, sondern durch je einen Linearmotor 16, 59 voneinander unabhängig angetrieben sind. Durch das Absaugen wird das verbrauchte CO₂-reiche Gas temporär aus dem Totraum entfernt und durch das über den zweiten Strang zugeführte Frischgas ersetzt.

Das vom ersten Strang 63 abgesaugte Gas wird am Ende der folgenden Einatmung bzw. zum Beginn der folgenden Ausatmung wieder zurückgeführt, während im Wesentlichen gleichzeitig vom zweiten Strang 64 frisches Gas aus dem Beatmungssystem abgepumpt wird.

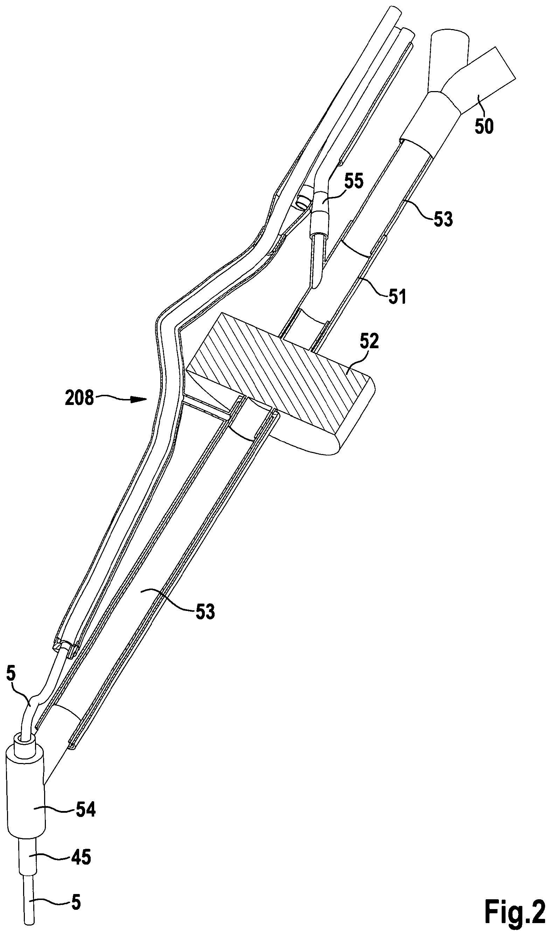

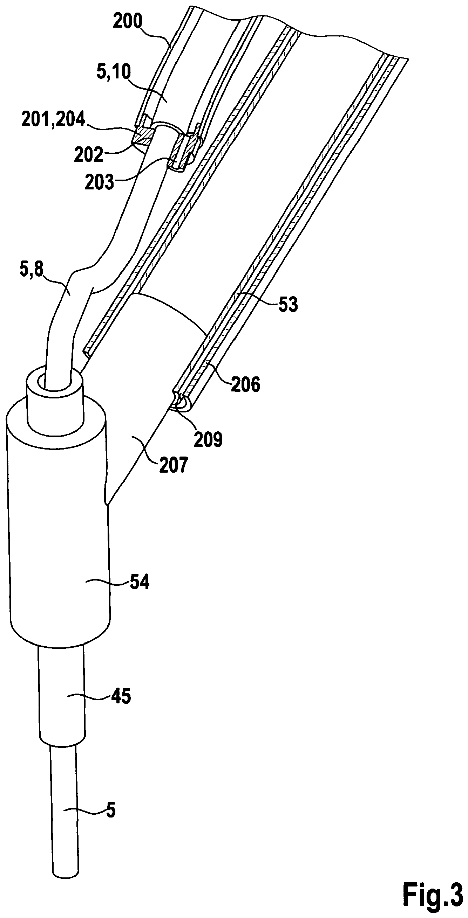

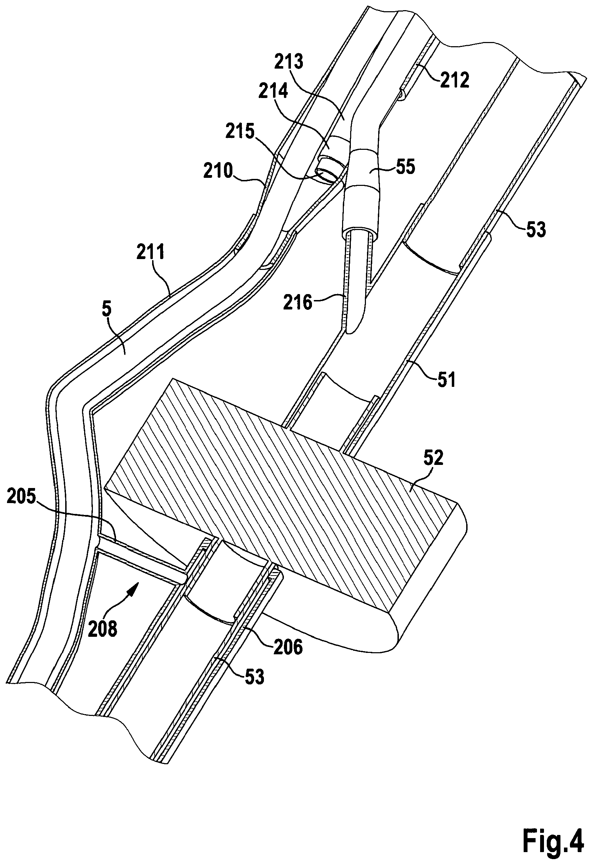

Während Figur 1 einen eher schematischen Überblick über das gesamte System 1 gibt, zeigen die Figuren 2 bis 4 etwas detaillierter den Bereich vom Tubus 45 bis zum Y-Stück 50, um die Temperierung des flexiblen Schlauchs 5 zu zeigen. Dazu sind einige weitere Bauteile dargestellt, die in Figur 1 zur besseren Übersicht nicht gezeigt sind.

Der flexible Schlauch 5 ist von einem Hüllschlauch 200 umgeben, der patientenseitig bis fast bis zum zweiten Konnektor 54 reicht. Der Hüllschlauch 200 weist an seinem patientenseitigen Ende ein Endstück 201 auf mit einer zentrischen Durchführöffnung 202 für den flexiblen Schlauch 5 sowie mit einer exzentrischen ersten Leckageöffnung 203. Das Endstück 201 bildet ein Anschlussstück 204, da es nicht nur den Abschluss des Hüllschlauchs 200 bildet, sondern auch der flexible Schlauch 5 vom ersten Schlauchabschnitt 8 an den zweiten Schlauchabschnitt 10 angeschlossen ist.

Nahe dem Filterelement 52 weist der Hüllschlauch 200 ein Verbindungsrohr 205 auf, das den Hüllschlauch 200 mit einem Hüllrohr 206 verbindet, das die Tubusverlängerung 53 mit einem Ringspalt umgibt. Im Bereich des Übergangs von der Tubusverlängerung 53 zu einem Anschlussstutzen 207 des zweiten Konnektor 54 endet das Hüllrohr 206. Der Anschlussstutzen 207 ist in die Tubusverlängerung 53 gesteckt. Das Verbindungsrohr 205 und das Hüllrohr 206 bilden zusammen eine Abzweigung 208. Da der Innendurchmesser des Hüllrohrs 206 größer als der Außendurchmesser des Anschlussstutzens 207 ist, bildet die Abzweigung 208 an diesem Ende des Hüllrohrs 206 eine zweite Leckageöffnung 209. Das gegenüberliegende Ende des Hüllrohrs 206 schließt im Bereich des Filters 52 dichtend mit der Tubusverlängerung 53 ab.

Vom Patienten weg gesehen schließt sich im weiteren Verlauf des Hüllschlauchs 200 ein Übergangsstück 210 an, durch das der Innen- und Außendurchmesser des Hüllschlauchs 200 erweitert wird. Das Übergangsstück 210 stellt also eine Stufung dar. Der Hüllschlauch 200 wird von einem ersten armierten, transparenten Wellschlauch 211 kleineren Durchmessers auf der einen Seite des Übergangsstücks 210 und einem zweiten armierten, transparenten Wellschlauch 212 größeren Durchmessers auf der anderen Seite des Übergangsstücks 210 gebildet. Durch den größeren Durchmesser ist es möglich, im zweiten Wellschlauch 212 weitere Schläuche unterzubringen. Insbesondere reicht ein Heizschlauch 213 bis zum Übergangsstück 210 und wird von einer ringförmigen Halteeinrichtung 214, die einstückig mit dem Übergangsstück 210 ist, klemmend gehalten, insbesondere gegen eine Verschiebung längs des Hüllschlauchs 200. Der Heizschlauch 213 weist im Bereich des Übergangsstücks 210 ein offenes Ende 215 auf. Weiterhin ist in dem Hüllschlauch 200 der weitere flexible Schlauch 55 bis zu dem Übergangsstück 210 und seitlich aus diesem herausgeführt. Das Ende des weiteren flexiblen Schlauchs 55 ist auf einen seitlich abstehenden Abzweigstutzen 216 des ersten Konnektors 51 gesteckt. In der gleichen Weise ist der dritte flexible Schlauch 65 bis zum Übergangsstück 210 und zum ersten Konnektor 51 geführt, was zur besseren Übersicht jedoch nicht dargestellt ist.

Am patientenfernen Ende der flexiblen Schläuche 5, 55, 65 sind die oben beschriebenen Aggregate, also insbesondere die Kolbenpumpen 14, 56, Ventile etc., in einem nicht dargestellten Gehäuse zusammengefasst. Der Hüllschlauch 200 reicht bis zu diesem Gehäuse. Der Heizschlauch 213 ist an ein Heizaggregat und einen Lüfter (beides nicht dargestellt) angeschlossen, wobei als Wärmequelle gegebenenfalls die Kolbenpumpen 14, 56, die Steuerung 37 oder andere Elemente, zumindest ergänzend, dienen können. Von dem Lüfter wird warme Luft als Medium zum Heizen in den Heizschlauch 213 geblasen. Am offenen Ende 215 des Heizschlauchs 213 verlässt die Luft den Heizschlauch 213. Ein Teil der Luft fließt in entgegengesetzte Richtung wieder zurück und zwar außerhalb des Heizschlauchs 213 und innerhalb des Hüllschlauchs 200. Hierdurch werden die flexiblen Schläuche 5, 55, 65 temperiert. Ein anderer Teil der Luft fließt weiter innerhalb des ersten Wellschlauchs 211 entlang des flexiblen Schlauchs 5 und zwar einerseits bis zur ersten Leckageöffnung im Endstück 201 und andererseits bis zur zweiten Leckageöffnung 209 in der Abzweigung 208, wo die Luft jeweils entweicht. Hierdurch wird der flexible Schlauch 5 und darüber hinaus die Tubusverlängerung 53 temperiert.

Zur Steuerung dieser Temperierung ist sowohl am gehäuseseitigen Ende des Heizschlauchs 213 als auch am gehäuseseitigen Ende des Hüllschlauchs 200, dort wo die zurückströmende Luft austritt, jeweils ein Temperatursensor angeordnet (nicht dargestellt). Die Temperatursensoren sind an die Steuerung 37 angeschlossen. Weiterhin können Druck- und Durchflusssensoren vorgesehen sein, um den Anteil der austretenden und der rückfließenden Luft messen, beziehungsweise überwachen, zu können.

Alternativ zur Abzweigung 208 kann beispielsweise ab dem Übergangsstück 210 statt des ersten Wellschlauchs 211 ein beutelartiger Schlauch vorgesehen werden (nicht dargestellt), der neben dem flexiblen Schlauch 5 auch die Tubusverlängerung 53 und weitere Bauteile, wie den ersten Filter 22, enthalten kann. Auch dieser beutelartige Schlauch weist mindestens eine Leckageöffnung auf, damit Luft zum Temperieren der Bauteile nachfließen kann.

### Bezugszeichenliste

- 1: System
- 2: Patient
- 4: Hals
- 5: Flexibler Schlauch
- 6: Luftröhre
- 7: Lunge
- 8: Erster Schlauchabschnitt
- 9: Katheter
- 10: Zweiter Schlauchabschnitt
- 11: Verbindungsschlauch
- 12: Reservoireinheit
- 14: Kolbenpumpe
- 15: Pumpeinheit
- 16: Linearmotor
- 17: Antriebseinheit
- 18: Messküvette
- 20: CO2-Sensor
- 21: Erster Abzweig
- 22: Erster Filter
- 23: Zweiter Abzweig
- 24: Hochdrucksensor
- 25: Be- und Entlüftungsventil
- 34: Heizelement
- 35: Temperiereinheit
- 37: Steuerung
- 38: Berührungsempfindliche Anzeige
- 44: Benutzerschnittstelle
- 45: Tubus
- 46: Leitungssystem des Beatmungssystems 47
- 47: Beatmungssystem
- 48: Zentraleinheit des Beatmungssystems 47
- 49: Beatmungsschlauch
- 50: Y-Stück
- 51: Erster Konnektor
- 52: Filterelement
- 53: Tubusverlängerung
- 54: Zweiter Konnektor
- 55: Weiterer flexibler Schlauch
- 56: Weitere Kolbenpumpe
- 57: Weitere Pumpeinheit
- 58: Weitere Reservoireinheit
- 59: Weiterer Linearmotor
- 60: Weitere Temperiereinheit
- 61: Weiterer Hochdrucksensor
- 62: Weiteres Be- und Entlüftungsventil
- 63: Erster Strang
- 64: Zweiter Strang
- 65: Dritter flexibler Schlauch
- 66: Beatmungsdrucksensor
- 67: Kommunikationsschnittstelle
- 200: Hüllschlauch
- 201: Endstück
- 202: Durchführöffnung
- 203: Erste Leckageöffnung
- 204: Anschlussstück
- 205: Verbindungsrohr
- 206: Hüllrohr
- 207: Anschlussstutzen
- 208: Abzweigung
- 209: Zweite Leckageöffnung
- 210: Übergangsstück
- 211: Erster Wellschlauch
- 212: Zweiter Wellschlauch
- 213: Heizschlauch
- 214: Halteeinrichtung
- 215: Offenes Ende des Heizschlauchs 213
- 216: Abzweigstutzen des ersten Konnektors 51

## Patentansprüche

1. System (1) zur Unterstützung des pulmonalen Gasaustauschs bei Patienten (2) und zur Kopplung an ein Beatmungssystem (47) oder zur Anwendung bei nicht-beatmeten Patienten (2), das einen, insbesondere in die Luftröhre (6) eines Patienten (2) einführbaren, flexiblen Schlauch (5), eine bidirektional wirkende Pumpeinheit (15) und insbesondere eine Steuerung (37) derart aufweist, dass über den flexiblen Schlauch (5) mittels der Pumpeinheit (15) eine, insbesondere end-exspiratorische, Absaugung und eine, insbesondere endinspiratorische, Rückführung des abgesaugten Gases einstellbar ist, wobei der flexible Schlauch (5) zumindest abschnittsweise von einem Hüllschlauch (200) umfasst ist, wobei innerhalb des Hüllschlauchs (200) außerdem ein Heizschlauch (213) zum Führen eines Mediums angeordnet ist, derart, dass das Medium durch den Heizschlauch (213) in eine erste Richtung und zumindest teilweise außerhalb des Heizschlauchs (213) sowie innerhalb des Hüllschlauchs (200) in eine entgegengesetzte zweite Richtung führbar ist, **dadurch gekennzeichnet, dass** das Medium derart führbar ist, dass es zunächst die Pumpeinheit (15) beheizt und dann erst in den Heizschlauch (213) geleitet wird.

2. System nach Anspruch 1, **dadurch gekennzeichnet, dass** der Heizschlauch (213) innerhalb des Hüllschlauchs (200) ein offenes Ende (215) und/oder Öffnungen aufweist

3. System nach Anspruch 1 oder 2, **dadurch gekennzeichnet, dass** am oder im Hüllschlauch (200) eine Halteeinrichtung (214) zum Halten des Heizschlauchs (213) in einer festen Position längs des Hüllschlauchs (200) aufweist.

4. System nach einem der vorstehenden Ansprüche, **dadurch gekennzeichnet, dass** der Hüllschlauch (200) oder ein mit dem Hüllschlauch (200) verbundenes Anschlussstück (204) eine Leckageöffnung (203) aufweist derart, dass nach dem Austritt des Mediums aus dem Heizschlauch (213) ein Teil des Mediums aus dem Hüllschlauch (200) oder dem Anschlussstück (204) austreten kann.

5. System nach einem der vorstehenden Ansprüche, **dadurch gekennzeichnet, dass** der Hüllschlauch (200) armiert, zweischichtig und/oder ein Wellschlauch (211, 212) ist.

6. System nach einem der vorstehenden Ansprüche, **dadurch gekennzeichnet, dass** der Hüllschlauch (200) transparent ist.

7. System nach einem der vorstehenden Ansprüche, **dadurch gekennzeichnet, dass** innerhalb des Hüllschlauchs (200) ein weiterer flexibler Schlauch (55, 65) angeordnet ist.

8. System nach Anspruch 7, **dadurch gekennzeichnet, dass** der Hüllschlauch (200) eine Abzweigung (208) aufweist.

9. System nach einem der vorstehenden Ansprüche, **dadurch gekennzeichnet, dass** ein Temperatursensor derart angeordnet ist, dass die Temperatur des Mediums im Bereich des Anfangs und/oder Endes des Wegs des Mediums messbar ist.

10. System nach einem der vorstehenden Ansprüche, **dadurch gekennzeichnet, dass** der Hüllschlauch (200), der Heizschlauch (213) und/oder der flexible Schlauch (5) Abschnitte mit unterschiedlichen Außendurchmessern aufweist.

11. System nach einem der vorstehenden Ansprüche, **dadurch gekennzeichnet, dass** der Hüllschlauch (200) einen Filter, einen Sensor und/oder eine Tubusverlängerung (53) umhüllt, um das jeweilige Element zusätzlich zum flexiblen Schlauch (5) zu temperieren.

## Claims

1. System (1) for assisting pulmonary gas exchange in patients (2) and for coupling to a ventilation system (47) or for use in non-ventilated patients (2), which system comprises a flexible tube (5), which can in particular be inserted into the trachea (6) of a patient (2), a bidirectionally acting pump unit (15), and in particular a controller (37), such that an in particular end-expiratory extraction and an in particular end-inspiratory return of the extracted gas can be adjusted via the flexible tube (5) by means of the pump unit (15), the flexible tube (5) being surrounded, at least in portions, by an outer tube (200), a heating tube (213) for conducting a medium also being arranged within the outer tube (200), such that the medium can be conducted through the heating tube (213) in a first direction, and at least partially outside the heating tube (213) and inside the outer tube (200) in an opposite second direction, **characterized in that** the medium can be conducted in such a way that it first heats the pump unit (15) and only then is conveyed into the heating tube (213).

2. System according to claim 1, **characterized in that** the heating tube (213) has an open end (215) and/or openings within the outer tube (200)

3. System according to either claim 1 or claim 2, **characterized in that** has a holding device (214) on or in the outer tube (200) for holding the heating tube (213) in a fixed position along the outer tube (200).

4. System according to any of the preceding claims, **characterized in that** the outer tube (200) or a connection piece (204) connected to the outer tube (200) has a leakage opening (203), such that after the medium has been discharged from the heating tube (213), a portion of the medium can escape from the outer tube (200) or the connection piece (204).

5. System according to any of the preceding claims, **characterized in that** the outer tube (200) is reinforced, has two layers and/or is a corrugated tube (211, 212).

6. System according to any of the preceding claims, **characterized in that** the outer tube (200) is transparent.

7. System according to any of the preceding claims, **characterized in that** a further flexible tube (55, 65) is arranged within the outer tube (200).

8. System according to claim 7, **characterized in that** the outer tube (200) has a branch (208).

9. System according to any of the preceding claims, **characterized in that** a temperature sensor is arranged such that the temperature of the medium can be measured in the region of the start and/or end of the path of the medium.

10. System according to any of the preceding claims, **characterized in that** the outer tube (200), the heating tube (213) and/or the flexible tube (5) has portions having different outer diameters.

11. System according to any of the preceding claims, **characterized in that** the outer tube (200) surrounds a filter, a sensor and/or a tube extension (53) in order to control the temperature of the relevant element in addition to that of the flexible hose (5).

## Revendications

1. Système (1) destiné à l'assistance à l'échange gazeux pulmonaire chez des patients (2) et à l'accouplement à un système ventilatoire (47) ou à l'utilisation chez des patients (2) non ventilés, lequel système présente un tuyau flexible (5) pouvant être inséré en particulier dans la trachée (6) d'un patient (2), une unité de pompage (15) à action bidirectionnelle et en particulier un dispositif de commande (37), de telle sorte qu'une aspiration, en particulier en fin d'expiration, et un renvoi, en particulier en fin d'inspiration, du gaz aspiré peuvent être réglés par l'intermédiaire du tuyau flexible (5) au moyen de l'unité de pompage (15), dans lequel le tuyau flexible (5) est entouré, au moins dans certaines sections, par un tuyau d'enveloppe (200), dans lequel un tuyau de chauffage (213) est en outre disposé à l'intérieur du tuyau d'enveloppe (200) pour le guidage d'un milieu, de telle sorte que le milieu peut être guidé à travers le tuyau de chauffage (213) dans un premier sens et au moins partiellement à l'extérieur du tuyau de chauffage (213) ainsi qu'à l'intérieur du tuyau d'enveloppe (200) dans un second sens opposé, **caractérisé en ce que** le milieu peut être guidé de telle sorte qu'il est d'abord chauffé par l'unité de pompage (15) et seulement ensuite dirigé dans le tuyau de chauffage (213).

2. Système selon la revendication 1, **caractérisé en ce que** le tuyau de chauffage (213) présente une extrémité ouverte (215) et/ou des ouvertures à l'intérieur du tuyau d'enveloppe (200)

3. Système selon la revendication 1 ou 2, **caractérisé en ce que** présente, sur ou dans le tuyau d'enveloppe (200), un appareil de maintien (214) pour le maintien du tuyau de chauffage (213) dans une position fixe le long du tuyau d'enveloppe (200).

4. Système selon l'une des revendications précédentes, **caractérisé en ce que** le tuyau d'enveloppe (200) ou une pièce de raccordement (204) reliée au tuyau d'enveloppe (200) présente une ouverture de fuite (203) de telle sorte qu'après la sortie du milieu hors du tuyau de chauffage (213), une partie du milieu peut sortir hors du tuyau d'enveloppe (200) ou de la pièce de raccordement (204).

5. Système selon l'une des revendications précédentes, **caractérisé en ce que** le tuyau d'enveloppe (200) est armé, est à deux couches et/ou est un tuyau ondulé (211, 212).

6. Système selon l'une des revendications précédentes, **caractérisé en ce que** le tuyau d'enveloppe (200) est transparent.

7. Système selon l'une des revendications précédentes,
**caractérisé en ce qu'un** autre tuyau flexible (55, 65) est disposé à l'intérieur du tuyau d'enveloppe (200).

8. Système selon la revendication 7, **caractérisé en ce que** le tuyau d'enveloppe (200) présente une ramification (208).

9. Système selon l'une des revendications précédentes,
**caractérisé en ce qu'**un capteur de température est disposé de telle sorte que la température du milieu peut être mesurée dans la zone du début et/ou de la fin du trajet du milieu.

10. Système selon l'une des revendications précédentes, **caractérisé en ce que** le tuyau d'enveloppe (200), le tuyau de chauffage (213) et/ou le tuyau flexible (5) présentent des sections comportant des diamètres extérieurs différents.

11. Système selon l'une des revendications précédentes, **caractérisé en ce que** le tuyau d'enveloppe (200) enveloppe un filtre, un capteur et/ou une prolongation tubulaire (53) afin de réguler la température de l'élément respectif en plus de celle du tuyau flexible (5).
